# EUROPEAN PATENT APPLICATION

(11) **EP 3 699 171 A1**
(43) Date of publication of application: **26.08.2020**
(21) Application number: 20166481.0
(22) Date of filing: 16.12.2014
(51) Int. Cl.: C07C 381/04, A61K 31/185, A61P 37/02

(54) **A PROPYL PROPANE THIOSULFONATE (PTSO) PRODUCT FOR USE AS AN IMMUNOMODULATOR IN HUMANS**

(30) Priority: 26.02.2014 US 201414191158
(62) Divisional of application: 14883542.4
(71) Applicant: Investfood, LLC, 18620 Granada (ES)
(72) Inventor: BAÑOS ARJONA, Alberto, 18620 Alhendin (ES); GÁLVEZ PERALTA, Julio J., 18620 Alhendin (ES); GUILLAMÓN AYALA, Enrique, 18620 Alhendín (ES); NÚÑEZ LECHADO, Cristina, 18620 Alhendín (ES); MAROTO CABA, Francisco, 18620 Alhendín (ES); RODRÍGUEZ CABEZAS, María Elena, 18620 Alhandín (ES)
(74) Representative: Sahuquillo Huerta, Jesús

(57) **Abstract**

The present invention relates to a propyl propane thiosulfonate (PTSO) formulation for modulating the immune response in healthy or sick individuals. The formulation includes selecting a compound based on PTSO. The compound based on PTSO contains PTSO the formula of which is R-SOa-S-R, where "R" represents the n-propyl (-CH2-CH2-CH3) group and "a" is 2. The compound based on PTSO is then incorporated into a PTSO formulation.

## Description

The present invention relates to immunology, and more specifically to modulating the immune response in humans.

### BACKGROUND OF THE INVENTION

Immunology is the biomedical science concerned with the study of all the aspects related with the body's natural defence system. Normal functioning of the immune system is crucial for human health. It is possible to intervene in regulating immune response by means of applying certain substances. Immunomodulation refers to the action exerted by given mediators on autoregulation processes that guide the immune defence system. Many compounds, such as azathioprine, 6-mercaptopurine, methotrexate, cyclosporine, levamisole and thalidomide are used as immunomodulators, being capable of optimizing the immune response, improving the general state of health and reducing the risk of suffering a given disease.

Immunomodulators can act like immunosuppressants or like immunostimulants by means of inhibiting or activating the immune response. Immunosuppressants help in treating inflammatory and autoimmune diseases, whereas immunostimulants can improve the immune function of people with chronic infectious diseases, immunodeficiency disorders, or even cancer.

Immunomodulators can act on two levels of the immune system, either by modulating the specific immune response by means of increasing immunoglobulin synthesis, or by modulating the innate immune response through regulation of the production of soluble mediators. In the first case, the immunomodulator exerts an immune reaction to a specific antigen, as occurs with the immunological adjuvants. These are chemical or biological preparations making the immune response more effective, making it faster, stronger and longer lasting. Immunological adjuvants play a decisive role in the process of inducing immune response by means of activating and recruiting accessory cells and other co-stimulators. In the innate immune response, immunomodulators can cause the stimulation or suppression of said response without requiring prior activation by a given antigen, as occurs in cytokine synthesis regulation.

Cytokines are a varied group of peptides, proteins or glycoproteins that act as hormonal regulators in the body. They play an essential role in the innate immune response by means of regulating defence mechanisms against invading agents or by means of mobilizing immunoregulatory processes, such as initiation of the inflammatory response or activation of immunocompetent cells. Some of the main cytokines involved in the innate immune response are interleukin-1, interleukin-6, interleukin-8, tumour necrosis factor (TNF-α) or interferon.

There is a growing need today in clinical practice or in the functional nutrition sector for the development of new, stronger, safer immunomodulatory compounds that can develop a safe and effective immune response.

### DISCLOSURE OF THE INVENTION

The present invention relates to a therapeutic composition that offers a novel and non-obvious method for obtaining a product for modulating the immune function in humans. The PTSO compound has formula R-SOa-S-R, where "R" represents the n-propyl (-CH2-CH2-CH3) group and "a" is 2. When the compound based on PTSO is selected, the compound based on PTSO can be incorporated into the PTSO product. A dose of the PTSO product can include an amount of compound based on PTSO between at least 0.01 mg and not more than 2,000 mg.

In another form of application, an amount of PTSO can be selected for forming a compound based on PTSO. When the compound based on PTSO is selected, the compound based on PTSO can be incorporated into the PTSO product. A dose of the PTSO product can include an amount of compound based on PTSO between at least 0.01 mg and not more than 2,000 mg.

Propyl propane thiosulfonate (PTSO) with the formula R-SOa-S-R, where "R" represents the n-propyl (-CH2-CH2-CH3) group and "a" is 2, can be administered to humans for the purpose of modulating the immune response.

In another form of application, a PTSO product (and, therefore, a compound based on PTSO) can be administered to humans to treat and/or prevent chronic infectious diseases, immunodeficiency disorders, cancer, a number of inflammatory bowel diseases, as well as other chronic autoimmune or inflammatory conditions or diseases.

Other additional aspects of the invention will be partially described in the following description and will be partially left out in said description, or they may be discovered when putting the invention into practice. The aspects of the invention will be carried out and reached by means of using elements and specific combinations described in the attached claims. It must be understood that both the preceding general description and the following detailed description are merely exemplary and explanatory and are not restrictive for the invention as claimed.

### DESCRIPTION OF THE DRAWINGS

The attached drawings that are incorporated into and part of this description illustrate the embodiments of the invention and, together with the description, explain the principles of the invention. The embodiments illustrated herein are the currently preferred embodiments. It must be understood, however, that the invention is not limited to the exact arrangement or to the instruments shown, such that:
Figure 1 shows a pictorial illustration of a method for modulating the immune response in humans.
Figure 2A shows a flowchart illustrating a method for modulating the immune function in humans.
Figure 2B shows a flowchart illustrating the use of PTSO as an immunomodulatory agent in another application of the invention.
Figure 2C shows a flowchart illustrating the use of PTSO as an immunomodulatory agent in another application of the invention.
Figure 3 shows a bar graph illustrating the effect of PTSO on modulation of IL-1β production in a dose-dependent manner.
Figure 4 illustrates the effect of PTSO on modulation of the IL-6 production.
Figure 5 illustrates the immunomodulatory activity of PTSO in the production of the cytokine IL-8 in human colon adenocarcinoma (CACO-2) cells.
Figure 6 illustrates modulation of interferon gamma (IFN-γ) production in a human leukaemia cell line.

### DETAILED DISCLOSURE OF THE INVENTION

The present invention describes a method for the application of an immunomodulatory compound. Specifically, in a form of application of the invention, a PTSO product can be used by selecting an amount of PTSO to yield a compound based on PTSO which will be incorporated into a PTSO product. The compound based on PTSO can include PTSO with the formula R-SOa-S-R, where "R" represents the n-propyl (-CH2-CH2-CH3) group and "a" is 2. The compound based on PTSO can then be incorporated into a PTSO product to create the compound based on PTSO containing a selected amount of PTSO. The selected amount of PTSO will be the amount required to have a dose of PTSO product (in each application) containing an amount of compound based on PTSO between at least 0.01 mg and not more than 2,000 mg. The PTSO product may be administered to humans as an immunomodulatory agent to improve the immune response in different pathological processes for the purpose of preventing and treating chronic infectious diseases, immunodeficiency disorders, cancer and systemic inflammatory diseases and inflammatory bowel diseases.

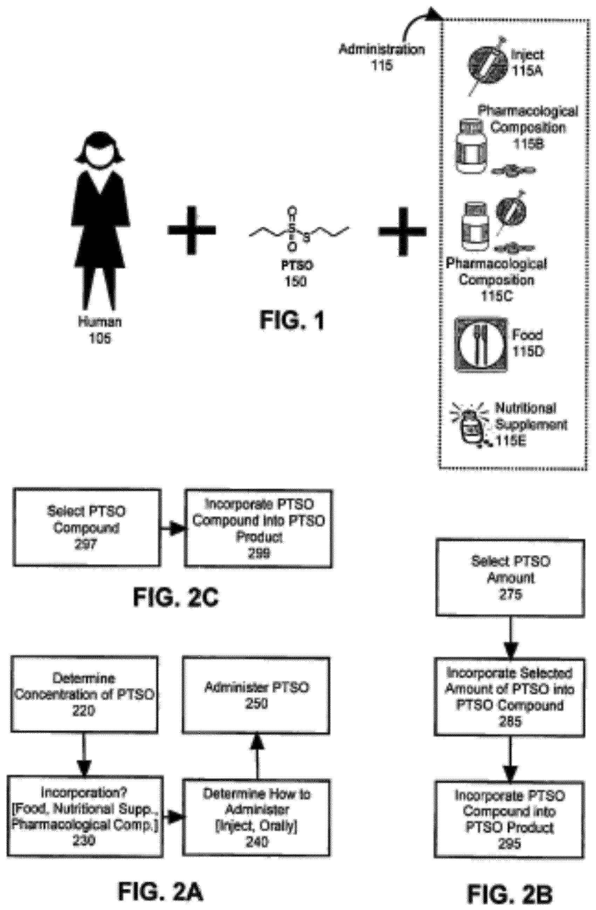

In the additional illustration, Figure 1 graphically represents the process of modulating the immune response. As shown in Figure 1, PTSO 150 can be administered to a healthy or sick individual 105. The administration 115 of PTSO 150 (and hence of a compound based on PTSO via a PTSO product) may be by means of an injection 115A, incorporated into a pharmacological formulation for oral administration 115B, incorporated into a pharmacological formulation for parenteral administration 115C, by means of the incorporation into a product and/or food supplement 115D, or by means of the incorporation into a nutritional supplement 115E. After the continuous administration of PTSO 150, there will be an improvement in the immune response in humans.

Figure 2A shows a flowchart illustrating the process whereby the immune function in humans is modulated. Starting with block 220, the desired concentration of PTSO in a compound based on PTSO for administration thereof is determined. The dose of PTSO product can contain an amount of compound based on PTSO between at least 0.01 mg and not more than 2,000 mg. In a different form of application of the invention, the dose may be comprised between 0.01 and 1.000 mg. In another different, additional form of application, the dose may be comprised between 0.5 and 550 mg.

As illustrated in block 230, a decision can be made as regards incorporating the compound based on PTSO (and hence PTSO itself) into a PTSO product such as a food and/or a food supplement, nutritional supplement and/or pharmacological formulations. It must be mentioned that the compound based on PTSO can be administered by itself (only PTSO, without other additives). A decision can then be made as to how to administer the compound based on PTSO (and, therefore, a PTSO product), as shown in block 240. For example, the compound based on PTSO can be injected or administered orally. After that point, the compound based on PTSO can be administered, as shown in block 250, for the purpose of modulating the immune function in individuals to treat and/or prevent chronic infectious diseases, immunodeficiency disorders, cancer, systemic inflammatory diseases or inflammatory bowel diseases, and chronic and autoimmune inflammatory conditions and diseases.

PTSO corresponds to the following formula:

R-SOa-S-R

where "R" represents the n-propyl (-CH2-CH2-CH3) group and "a" is 2. It must be mentioned that PTSO is also known as dipropyl propyl thiosulfonate, propyl propane thiosulfonate or 1-propylsulfonylsulfanylpropane (CAS number 1113-13-9).

It must be mentioned that in addition to modulation of the immune function, which can include both modulation of cytokine production and production of other markers, it is also contemplated that the invention can be used for preventing or treating chronic infectious diseases, such as immunodeficiency disorders, such as acquired immunodeficiency syndrome (AIDS), cancer, a number of inflammatory bowel diseases and other chronic and autoimmune diseases. Inflammatory diseases can include, but are not limited to, inflammatory bowel diseases (e.g. ulcerative colitis and Crohn's disease), ileitis, irritable bowel syndrome, Celiac disease and the chronic inflammatory bowel disease. In addition, it is contemplated in the present invention that PTSO can be used as an immunostimulatory agent. Specifically, PTSO can be used to potentiate the immune response against an antigen and/or to modulate said response towards a desired immune response (e.g. to improve the pre-existing immunity, increase specific immunoglobulin synthesis after exposure to an antigen, and/or increase specific immune response when used in combination with vaccinations or as an immunological adjuvant).

It must furthermore be mentioned that PTSO can be used by itself or in combination with other immunomodulatory products and/or pharmacological excipients. Depending on the dose of application, PTSO can exert an immunostimulatory or immunosuppressive effect. For example, at doses of 0.1 mM, the compound can increase IL-6 production in THP-1 cells whereas, in contrast, at doses exceeding 1 mM, the compound exerts a clear suppressive effect on synthesis of this interleukin. However, the dose-response ratio can specifically depend on the type of cytokine and/or the cell model studied.

Furthermore, PTSO can modulate the immune response in the presence of a given pathology and improve the immune response in healthy individuals. PTSO can be administered to a healthy or sick individual orally, either directly incorporated into a food and/or food supplement, nutritional supplement or into pharmacological formulations (including other immunomodulatory drugs and products). The application of the invention for oral administration thereof can be adopted under a presentation in the form of capsules, pills, tablets, powders, granulates or as an emulsion or a liquid solution in suspension (beverage, thick juice or syrup). Additionally, PTSO can be administered to an individual by means of an injection by itself or as a part of pharmacological formulation.

In another additional illustration of the invention, Figure 2B shows a flowchart illustrating the application of a PTSO product for modulating the immune response in humans. In block 255, an amount of PTSO can be selected. The selected amount of PTSO corresponds with a dose that is effective in creating a compound based on PTSO resulting in a dose of PTSO product between at least 0.01 mg and not more than 2,000 mg. In other words, a dose of PTSO product contains an amount of product based on PTSO between at least 0.01 mg and not more than 2,000 mg.

The selected amount of PTSO can be incorporated into a compound based on PTSO for the purpose of creating a compound based on PTSO at a desired concentration, as shown in block 285. As illustrated in block 295, the compound based on PTSO can be incorporated into a PTSO product. The PTSO product may be incorporated into any of the formulations suitable for oral administration thereof. For example, a PTSO product can be presented in the form of capsules, pills, powders and/or granulates. The PTSO product can also be presented in the form of a liquid (aqueous or non-aqueous) solution, an emulsion, a beverage and/or a syrup. Furthermore, the PTSO product can be presented as a pre-filled syringe. The PTSO solution can contain a compound based on PTSO with a selected concentration that can be used for a PTSO product in which the dose of PTSO product contains an amount of compound based on PTSO between at least 0.01 mg and not more than 2,000 mg. Given that the PTSO product is going to be administered to humans, it should be mentioned that it is a food-grade molecule.

In another additional illustration of the invention, Figure 2C shows a flowchart illustrating the application of a PTSO product for modulating the immune response in humans. As shown in block 297, a compound based on PTSO can be selected from a set of compounds based on PTSO that have already been produced. The compound based on PTSO can be incorporated into a PTSO product, as illustrated in block 299. Depending on the selected compound based on PTSO, the PTSO product can include an amount of the compound based on PTSO of at least 0.01 mg and not more than 2,000 mg. As indicated herein, the PTSO product can be presented in the form of capsules, pills, tablets, powders and/or granulates. The PTSO product can also be presented in the form of a liquid (aqueous or no-aqueous) solution, an emulsion, a beverage and/or a syrup. Furthermore, the PTSO product can be presented as a pre-filled syringe in which the pre-filled syringe is filled with a PTSO solution. The PTSO solution can be made up of a compound based on PTSO with the desired concentration. Since the PTSO product will be administered to humans and will be consumed by humans, the molecule is suitable for food use.

It must be mentioned that, as described in the present invention, PTSO can be an active compound by itself which can be dissolved in a (aqueous or no-aqueous) solution and/or be combined with other ingredients for forming a compound based on PTSO. This compound based on PTSO can then be incorporated together with other immunomodulatory products, pharmacological excipients, etc., for forming a PTSO product (pill, pastille, beverage, etc.). It is accepted that immunomodulatory products, excipients, etc., can also be added as part of the compound based on PTSO. Additionally, dose is understood to be a specific amount of a drug or therapeutic agent taken each time.

The following examples are presented in additional illustrations of the invention. It must be mentioned, as will be understood by a person skilled in the art, that the invention is not limited exclusively to these examples.

### Example 1

The evaluation of the immunosuppressive activity of PTSO in a human monocytic leukaemia cell line (THP-1) is studied in this example.

The effects of different concentrations of PTSO in cytokine production (IL-1β and IL-6 production) on human cell lines (THP-1) activated with lipopolysaccharide (LPS) and with phorbol 12-myristate 14-acetate have been evaluated. It must be mentioned that it has been reported that cytokines IL-1β and IL-6 play a key role in the immune response in different inflammatory and/or immune conditions.

Human cell line THP-1 was obtained from the Unidad de Cultivos Celulares (Cell Culture Unit) of the Universidad de Granada (Granada, Spain) (ECACC reference number: 88081201). The cells were cultured in cell culture medium RPMI 1640 (Roswell Park Memorial Institute, Sigma, St Louis, MO, USA) supplemented with 10% foetal bovine serum (Sigma), 2 mM of glutamine and 0.05 mM of mercaptoethanol in a humidified atmosphere at 37°C with 5% carbon dioxide (CO2). The cells were cultured in 24-well plates at a density of 5 x 105 cells/ml and were preincubated for 24 hours with the different concentrations of the compound (starting from 0.1 to 25 µM of PTSO) and stimulated with 1 µg/ml of LPS and 1 µM of phorbol 12-myristate 14-acetate (PMA). After 24 hours, the supernatant was collected and centrifuged at 1.000 g for 10 minutes. It was then stored at -80°C for assessing IL-1β and IL-6 levels by means of enzyme-linked immunosorbent assay (ELISA) (GE Healthcare, Little Chalfont, UK).

### Example 2

The immunomodulatory effect of PTSO in human colon adenocarcinoma cells (CACO-2) is demonstrated in Example 2.

The effects of different concentrations of PTSO on interleukin 8 production (IL-8) in a human colorectal adenocarcinoma (CACO-2) cell line activated by IL-1β were evaluated. IL-8 participates in the neutrophilic infiltration associated with mucosal inflammatory processes.

The cell line was obtained from the Centro de Cultura Celular (Cell Culture Centre) of the Universidad de Granada (Granada, Spain) (ECACC reference number: 86010202). The cells were cultured in cell culture medium RPMI 1640 (Sigma) supplemented with 10% foetal bovine serum (Sigma), 2 mM of glutamine and 0.05 mM of mercaptoethanol in a humidified atmosphere at 37°C with 5% carbon dioxide (CO2). The cells were cultured in 24-well plates to confluence and preincubated for 24 hours with the different concentrations of the compound (starting from 0.1 to 25 µM of PTSO) and stimulated with 1 ng/nl of IL-1β. After 24 hours, the supernatant was collected and centrifuged at 1.000 g for 10 minutes and was then stored at-80°C for assessing IL-1β levels by means of enzyme-linked immunosorbent assay (ELISA) (GE Healthcare). The effects of the different concentrations of PTSO on cell viability were evaluated using the methylthiazol tetrazolium (MTT) assay (Promega).

### Example 3

The immunomodulatory effect of PTSO on a human leukaemia cell line was evaluated in this example.

The cell line used was JURKAT E6.1, obtained from the Centro de Cultivos Celulares of the Universidad de Granada (Granada, Spain) (ECACC reference number: 88042803). The T cells from the cell line were cultured in cell culture medium RPMI 1640 supplemented with 10% foetal bovine serum (Sigma), 2 mM of glutamine and 0.05 mM of mercaptoethanol in a humidified atmosphere at 37°C with 5% carbon dioxide (CO2). The cells were cultured in 24-well plates at a concentration of 500,000 cells/ml and preincubated for 2 and 24 hours with different concentrations of the compound (starting from 0.1 to 25 µM of PTSO). They were then stimulated with 40 nM of ionomycin and 0.5 µM of PMA. After 24 hours, the supernatant was collected and centrifuged at 1.000 g for 10 minutes, and it was then stored at -80°C for assessing IFN-γ levels by means of the ELISA method (GE Healthcare).

### Results of the examples

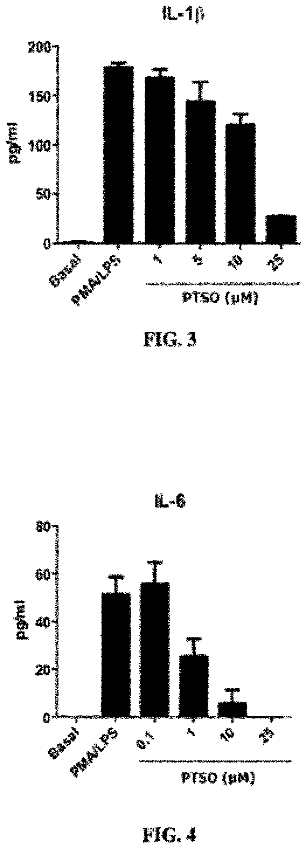

The results showed that PTSO could reduce IL-1β production in a dose-dependent manner as illustrated in Figure 3.

Figure 3 shows the effects of PTSO on the production of interleukin 1 beta (IL-1β), a pro inflammatory cytokine, which is a key mediator of the immune response, boosting the response of lymphocytes and regulating tissue injury. Some situations can lead the organism to produce an excessive amount of IL-1β to defend itself against external attacks, causing an adverse inflammatory reaction. In these situations, the use of compounds to reduce excess production of this cytokine is recommended in order to reduce inflammation. [[The definition of the cytokine is well-known prior art]]

Figure 3 illustrates the production of IL-1β in human cell lines (THP-1) stimulated with PMA/LPS, which are mitogens used to induce the cells to produce IL-1β. The first bar shows the Basal value, i.e. the amount of IL-1β produced by non-stimulated cells. The second bar represents the level of IL-1β production in cells stimulated with PMA/LPS (without adding PTSO). The other bars show the level of IL-1β production in cells stimulated with PMA/LPS in presence of increasing concentrations of PTSO. It is clearly observed that higher concentrations of PTSO (10 and 25 µM), reduce production of IL-1β. In summary, this graph shows the ability of PTSO to reduce IL-1β production in human cell lines stimulated with PMA/LPS. [[Written description of figure 3 as filed]]

Figure 4 illustrates the ability of PTSO to modulate IL-6 production.

Figure 4 shows the effect of PTSO on the production of interleukin 6 (IL-6), a cytokine that acts as either a pro-inflammatory or an anti-inflammatory cytokine, playing a crucial role as mediator of fever, and the acute phase response [[The definition of the cytokine is well-known prior art]]

Figure 4 illustrates the production of IL-6 in human cell lines (THP-1), stimulated with PMA/LPS, which are mitogens used to induce the cells to produce IL-6. The first bar shows the Basal value, i.e. the amount of IL-6 produced by non-stimulated cells (without adding PTSO). The second bar represents the level of IL-6 production in cells stimulated with PMA/LPS. The other bars show the level of IL-6 production in cells stimulated with PMA/LPS in presence of increasing concentrations of PTSO. A modulatory effect is observed depending on the dose of PTSO. In the third bar including 0.1 µM, PTSO produces an increase of IL-6. However, a dramatic decrease of this cytokine is clearly observed in higher concentrations, as shown in the bars indicating 10 and 25 µM PTSO. In summary, this graph shows the modulatory effect of PTSO for producing IL-6 depending on the applied dose in human cells stimulated with PMA/LPS. [[Written description of figure 4 as filed]]

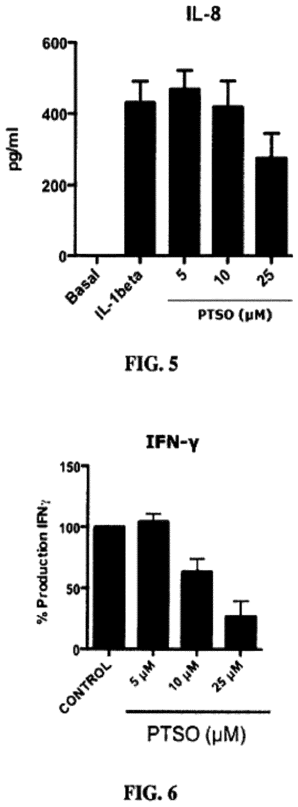

The immunomodulatory activity of PTSO in interleukin-8 production in human colon adenocarcinoma cells (CACO-2) has been demonstrated in Figure 5.

Figure 5 shows the effect of PTSO on the production of interleukin 8 (IL-8), a pro-inflammatory cytokine, which induces accumulation of neutrophils at sites of inflammation, although it can also lead to inflammatory processes in certain autoimmune diseases, such as rheumatoid arthritis.

Figure 5 illustrates the production of IL-8 in CACO-2 cell lines stimulated with IL 1 Beta, which, in this case, it acts as a mitogen to induce cells to produce IL-8. The first bar shows the Basal value, i.e. the amount of IL-8 produced by non-stimulated cells. The second bar shows the level of IL-8 production in cells stimulated with the mitogen (without adding PTSO). The other bars show the level of IL-8 production in cells stimulated with the mitogen in presence of increasing amounts of PTSO. A modulatory effect is observed depending on the dose of PTSO. In the third bar, including 0.1 µM, it is clearly shown that PTSO produces a slight increase of IL-8. However, increased concentrations show a significant reduction of this cytokine, as shown in the bar corresponding to 25 µM of PTSO. In short, this graph shows the modulatory effect of PTSO for producing IL-8 depending on the applied dose in human colon cells stimulated with IL-1 Beta.

Finally, Figure 6 illustrates modulation of the immune function of interferon gamma (IFN-γ) production in T cells from human leukaemia.

Figure 6 shows the effect of PTSO on the production of interferon-gamma (IFN-γ), a pro inflammatory cytokine. Production of IFN-γ to fight infections is a hallmark of immune response. However, its excessive release has been associated with the pathogenesis of chronic inflammatory and autoimmune diseases.

Figure 6 illustrates the production of IFN-γ in JURKAT cells. The first bar shows the Basal value, i.e. the amount of IFN-γ produced by cells. The other bars show the level of IFN γ cells production in presence of increasing concentrations of PTSO. At the lowest applied concentration of 5 µM PTSO, a slight increase of this cytokine is observed. However, higher concentrations of 10 and 25 µM PTSO show a decrease of IFN-γ. This figure shows the ability of PTSO to modulate the production of IFN-γ depending on the applied dose in JURKAT cells.

The viability assays clearly showed that concentrations of PTSO of up to 25 µM do not significantly affect THP-1, CACO-2 and JURKAT cell viability. Therefore, the effects clearly shown on cytokine production cannot be attributed to the action of the compound in cell viability. As a result, said effects demonstrate immunomodulatory activity of the compound independently of any cytotoxic effect.

Having described the invention in detail in the present application and having made reference to the embodiments thereof, it is obvious that modifications and variations are possible without departing from the scope of the invention, as defined in the following attached claims.

## Claims

1. A propyl propane thiosulfonate (PTSO) formulation for use as an immunomodulator of the innate immune response in humans without requiring prior activation by a given antigen as occurs in cytokine synthesis regulation
wherein the PTSO formulation comprises a PTSO based compound having a formula R-SOa-S-R, where "R" represents n-propyl group (-CH2-CH2-CH3) and "a" is 2.

2. The formulation for use as an immunomodulator according to claim 1 wherein PTSO formulation contains an amount of the PTSO based compound of at least 0.01 mg and not more than 2,000 mg.

3. The formulation for use as an immunomodulator according to claim 1 wherein the PTSO formulation contains an amount of the PTSO based compound of at least 0.01 mg and not more than 1,000 mg.

4. The formulation for use as an immunomodulator according to claim 1 wherein the PTSO formulation contains an amount of the PTSO based compound of at least 0.5 mg and not more than 550 mg.

5. The formulation for use as an immunomodulator according to claim 1 wherein the PTSO formulation is administered in a form selected between: pill, capsule, beverage, food supplement, nutritional supplement, a pharmacological formulation or a solution.

6. The formulation for use as an immunomodulator according to any of the claims 1 to 5 wherein the immunomodulator activity comprises the modulation of the cytokine IL-1β production.

7. The formulation for use as an immunomodulator according to any of the claims 1 to 5 wherein the immunomodulator activity comprises the modulation of the cytokine IL-6 production.

8. The formulation for use as an immunomodulator according to any of the claims 1 to 5 wherein the immunomodulator activity comprises the modulation of the cytokine IL-8 production.

9. The formulation for use as an immunomodulator according to any of the claims 1 to 5 wherein the immunomodulator activity comprises the modulation of the cytokine IFN-γ production.
